# EUROPEAN PATENT SPECIFICATION

(11) **EP 2 117 618 B1**
(45) Date of publication and mention of the grant of the patent: **03.11.2010**
(21) Application number: 07717009.0
(22) Date of filing: 23.01.2007
(51) Int. Cl.: A61L 31/02, C22C 14/00

(54) **IMPLANTABLE MEDICAL DEVICE WITH TITANIUM ALLOY HOUSING**
IMPLANTIERBARES MEDIZINPRODUKT MIT GEHÄUSE AUS EINER TITANLEGIERUNG
DISPOSITIF MÉDICAL IMPLANTABLE AVEC BOÎTIER EN ALLIAGE DE TITANE

(30) Priority: 31.10.2006 US 590678
(43) Date of publication of application: 18.11.2009
(73) Proprietor: Medtronic, Inc., Minneapolis, MN 55432-5604 (US)
(72) Inventor: LI, Bernard Q., Plymouth, Minnesota 55411 (US); GREVIOUS, John J., Minneapolis, Minnesota 55418 (US); DAVIS, Timothy J., Coon Rapids, Minnesota 55433 (US); PERZ, Leroy, Buffalo, Minnesota 55313 (US); PAIDOSH, Chris J., St. Anthony, Minnesota 55418 (US); KAST, John E., Hugo, Minnesota 55038 (US); MIESEL, Keith A., St. Paul, Minnesota 55104 (US); JANZIG, Darren A., Center City, Minnesota 55012 (US); LINDNER, Gerald G., Lino Lakes, Minnesota 55035 (US)
(74) Representative: Hughes, Andrea Michelle
(86) International application number: PCT/US2007/002042
(87) International publication number: WO 2008/054447

(56) References cited:
- EP-A- 0 534 782
- WO-A-01/97908
- DE-A1- 3 740 732
- US-A- 4 041 955
- US-A1- 2003 078 675
- KOMOTORI, J. ET AL: "Corrosion response of surface engineered titanium alloys damaged by prior abrasion" WEAR , 250-251(PT. 2), 1239-1249 CODEN: WEARAH; ISSN: 0043-1648, 2001, XP002449670

## Description

### BACKGROUND

The present invention relates generally to the field of implantable medical devices (IMDs) such as implantable neurological stimulation (INS) devices, drug pumps, and cardiac pacemakers. More particularly, the present invention relates to implantable medical devices that include titanium alloy housings or casings.

Implantable medical devices typically include external structures (e.g., housings or casings) that are made from biologically compatible materials to minimize undesirable interactions with the human body. One example of such a biologically compatible material that has been used for IMD housings is commercial pure titanium Grade 1 (hereinafter referred to as "CP Ti Grade 1"). This material has several characteristics that make it desirable for IMD housings, including its mechanical properties, which make it possible to form relatively small structures with complex geometries.

The use of CP Ti Grade 1 may not be optimal in all IMD applications, however. For example, certain IMDs may include batteries within their housings that are designed to be inductively charged while the IMDs are implanted. In such configurations, the IMD includes an electrically conductive coil or winding that is electrically coupled to the battery of the IMD. To charge the battery, a "primary" coil or winding from a charging system is placed near the location where the IMD is implanted and a current is sent through the primary coil; through induction, a current is then generated in the secondary coil that is transmitted to the battery.

Where the coil of the IMD is provided within the housing of the IMD, the CP Ti Grade 1 material may not be ideally suited to allow inductive charging. It may be desirable instead to form the housing from a material that exhibits greater power coupling efficiency and improved telemetry distance than would be possible if the structure of the device was made using CP Ti Grade 1. Additionally, because the IMD is typically subjected to various stresses during implantation and use, it may also be desirable to form the housing from a material that has greater strength than CP Ti Grade 1. Of note here is WO 01/97908

One alternative to CP Ti Grade 1 in the context of IMD housings is a titanium alloy having the formula Ti-6Al-4V (referred to as Ti64). Such an alloy has a greater tensile yield strength than CP Ti Grade 1 and also has better power coupling efficiency and improved telemetry distance. However, there are a number of disadvantages associated with the Ti-6Al-4V alloy. For example, it is relatively difficult to form thin sheets from Ti-6Al-4V alloy without subjecting the material to relatively high temperatures (e.g., approximately 850°C at its peak elongation). The use of such elevated temperatures may introduce increased complexity and cost into the forming operation, and may also produce undesirable oxidation of the titanium alloy. These limitations may make it relatively difficult to form relatively small structures having complex geometries using Ti-6Al-4V alloy. Of note here is US 4 041 955

It would be desirable to provide an implantable medical device that utilizes a material for its housing that allows for improved power coupling and telemetry distance, and which may have sufficient mechanical strength to provide enhanced protection for the device. It would also be desirable to provide an implantable medical device that utilizes a material that may be formed in a relatively simple and cost-efficient manner at relatively low temperatures. It would be desirable to provide an implantable medical device that includes any one or more of these or other advantageous features as will be apparent to those reviewing the present disclosure.

### SUMMARY

An exemplary embodiment of the invention relates to a rechargeable implantable medical device that includes a housing comprising a titanium alloy selected from the group consisting ofTi-4.5Al-3V-2Fe-2Mo-0.15O, Ti-4Al-2.5V-1.5Fe-0.25O, Ti-6Al-2Sn-4Zr-2Mo, Ti-3Al-2.5V, and combinations thereof.

Another exemplary embodiment of the invention relates to a rechargeable implantable neurological stimulation device that includes a housing formed from a titanium alloy selected from the group consisting of Ti-4.5Al-3V-2Fe-2Mo-0.15O, Ti-4Al-2.5V-1.5Fe-0.25O, Ti-6Al-2Sn-4Zr-2Mo, Ti-3Al-2.5V, and combinations thereof.

Another exemplary embodiment of the invention relates to a method of producing a rechargeable implantable medical device that includes forming a housing for an implantable medical device from a titanium alloy selected from the group consisting of Ti-4.5Al-3V-2Fe-2Mo-0.150, Ti-4Al-2.5V-1.5Fe-0.25O, Ti-6Al-2Sn-4Zr-2Mo, Ti-3Al-2.5V, and combinations thereof.

### BRIEF DESCRIPTION OF THE DRAWINGS

FIGURE 1 is a plan view of a housing or casing for an implantable medical device according to an exemplary embodiment.

FIGURE 2 is a graph illustrating the relationship between resistivity and tensile yield strength for a number of titanium alloys.

FIGURE 3 is a graph illustrating the relationship between resistivity and percent elongation for a number of titanium alloys.

FIGURE 4 is a contour plot illustrating the effect of varying amounts of aluminum and vanadium on the resistivity of titanium alloys.

FIGURE 5 is a contour plot illustrating the effect of varying amounts of aluminum and tin on the resistivity of titanium alloys.

FIGURE 6 is a graph illustrating a comparison of tensile yield strengths of various titanium alloys.

FIGURE 7 is a graph illustrating the relationship of percent elongation and stress versus temperature responses for two titanium alloys.

FIGURE 8 is a graph illustrating the relationship between strain rate and temperature responses for two titanium alloys.

FIGURE 9 is a graph illustrating the relationship between effective parallel coil resistance and sheet thickness for a number of titanium alloys.

FIGURE 10 is a graph illustrating the relationship between effective parallel coil resistance and resistivity for a number of titanium alloys.

FIGURE 11 is a graph illustrating the relationship between INS loss and current for two titanium alloys.

FIGURE 12 is a graph illustrating the relationship between charging frequency and coil coupling response for various titanium alloys.

### DETAILED DESCRIPTION

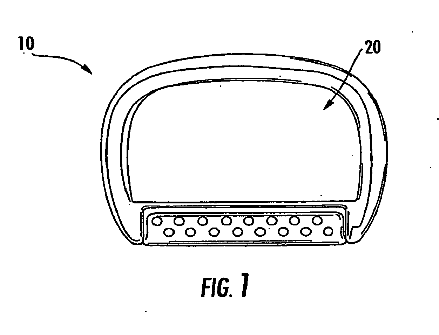

Referring to FIGURE 1, a housing or casing 20 for an implantable medical device (IMD) 10 in the form of an implantable neurological stimulation (INS) device is shown according to an exemplary embodiment. The housing 20 is intended to protect the IMD 10 from damage during implantation and use, and is formed of a biocompatible material to prevent undesirable interactions between the IMD 10 and the human body or other organism into which it is implanted. As will be described in greater detail below, the subject matter described herein may also find utility in other types of IMDs according to other exemplary embodiments, including drug pumps, cardiac pacemakers, and the like.

According to an exemplary embodiment, the IMD 10 includes a coil or winding (not shown) that is used to charge the IMD 10 by induction when another coil coupled to a charging device is provided proximate the IMD 10. In this manner, the IMD 10 may be recharged without the need to remove it from the body in which it is implanted (i.e., the charging may occur transdermally). According to an exemplary embodiment, the coil is provided inside the housing 20. According to other exemplary embodiments, the coil may be provided outside the housing.

According to an exemplary embodiment, the housing 20 is formed from a material that provides the IMD 10 with enhanced power coupling and recharging efficiency, improved telemetry distance, reduced heating effects during magnetic resonance imaging (MRI), and improved tolerance for compression stress as compared to devices that have housings or casing formed from CP Ti Grade 1. According to an exemplary embodiment, the entire housing 20 is made from the same material. According to other exemplary embodiments, a portion of the housing may be made from a different material (e.g., a different titanium alloy, etc.).

In selecting a titanium alloy for use as the housing 20, it is advantageous to select an alloy that has relatively high tensile yield strength (to provide improved strength of the housing) while still exhibiting relatively good formability at relatively low temperatures (to allow the manufacture of relatively small components having relatively complex geometries at temperatures between approximately 200° and 400°C, and more preferably at a temperature of approximately 500°C). One advantageous feature of using an alloy that exhibits relatively good formability at relatively low temperatures is that the occurrence of undesirable oxidation during the forming operation may be reduced or eliminated.

It is also advantageous to select an alloy that has a relatively high resistivity, since alloys having high resistivity will produce lower eddy currents and less energy loss in the form of heat loss than lower-resistivity alloys. In this manner, the charging of the implantable medical device may be made more efficient, since more of the charging energy will go toward charging the device.

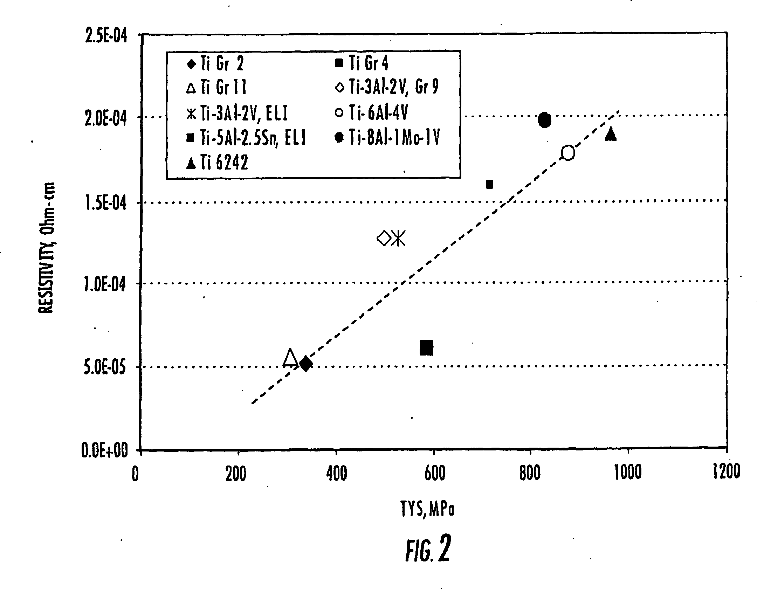

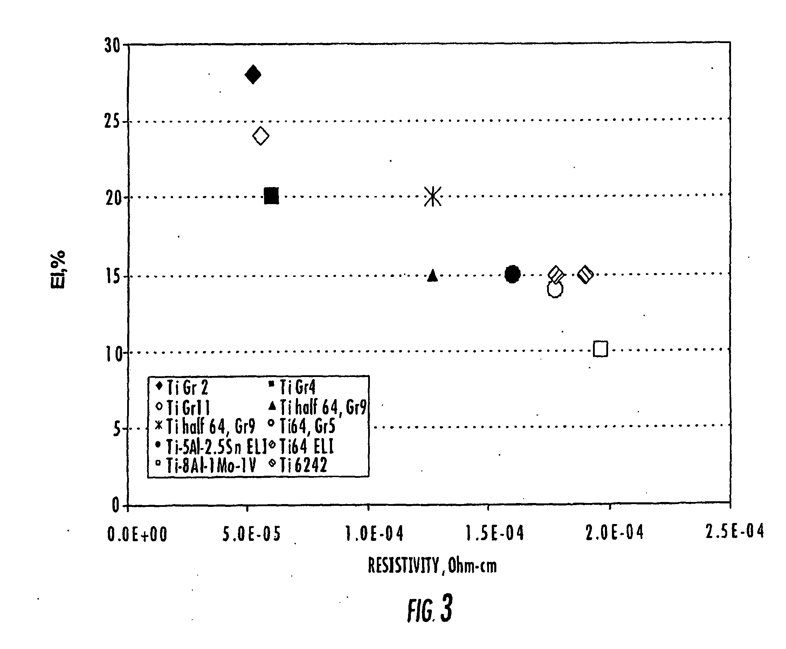

FIGURE 2 is a graph illustrating the relationship between resistivity and tensile yield strength for a number of titanium alloys, and FIGURE 3 is a graph illustrating the relationship between formability (shown as percent elongation of the alloys) and resistivity of such alloys. From this data, it is evident that resistivity of titanium alloys tends to increase with increasing tensile yield strength. Conversely, the resistivity tends to decrease with increasing formability.

It is generally understood that the tensile yield strength of titanium alloys generally increases with increasing alloy content. Based on this fact and the relationships illustrated in FIGURES 2 and 3, one would expect that titanium alloys having relatively high alloy content will have relatively high tensile yield strengths and resistivities, but will be less formable than alloys having lower alloy contents.

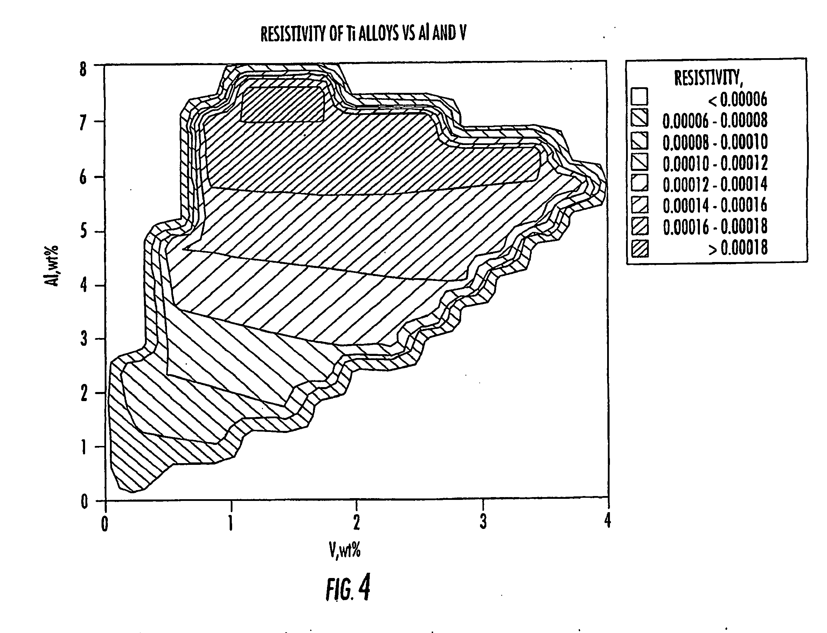

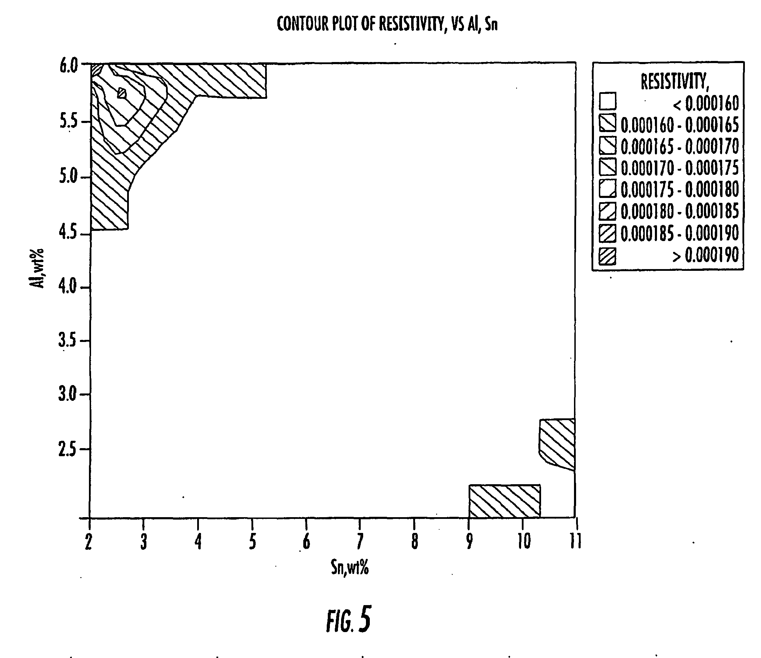

While the amount of alloying elements is understood to have an effect on the yield strength (and thus the resistivity) of the titanium alloys, different alloying elements have been shown to affect the resistivity of titanium alloys differently. For example, FIGURE 4 is a contour plot illustrating the change in resistivity for titanium-aluminum-vanadium alloys when the percentages of aluminum and vanadium are varied. In contrast, FIGURE 5 is a contour plot illustrating the change in resistivity for titanium-aluminum-tin alloys when the percentages of aluminum arid tin are varied. As can be seen from these contour plots, the effect on resistivity is different when the aluminum/vanadium percentages are varied as compared to the difference resulting from varying the aluminum/tin percentages.

According to an exemplary embodiment, the housing 20 is made from a titanium (Ti) alloy having the general formula

Ti-Al-B-C

where B represents one or more alloy elements such as V, Sn, Mo, Nb, Zr, and combinations thereof and C represents one or more impurity elements such as N, C, H, Fe, O, Si, Pd, Ru, and combinations thereof. Aluminum is provided in an amount between approximately 2 and 7 weight percent according to an exemplary embodiment. Elements represented by B and C in the above formula may be present in amounts shown in Table 1 according to various exemplary embodiments.

**Table 1**

| Element | Approximate Weight Percent |
|---|---|
| V | 2-6 |
| Sn | 1.5-6.5 |
| Mo | < 6 |
| Nb | < 2 |
| Zr | < 5 |
| N | < 0.05 |
| C | <0.1 |
| H | < 0.0015 |
| Fe | < 2 |
| O | < 0.3 |
| Si | <0.5 |
| Pd | < 1 |
| Ru | < 0.02 |

According to an exemplary embodiment, the alloy has a resistivity between approximately 100 µmΩ-cm and 210 µmΩ-cm and a tensile yield strength of between approximately 65 ksi and 150 ksi.

As described above, it is also beneficial for the alloy selected to have relatively good formability performance to allow the alloy to be formed into relatively thin sheets that may be formed into structures having relatively complex geometries (e.g., structures having relatively small features and sharp corners). For example, according to an exemplary embodiment, at least a portion of the housing (e.g., a "comer" of the housing) has a radius of curvature of between approximately 0.1 and 2.5 millimeters.

It should be noted that there are various tests available to characterize the relative formability of different alloys, including tensile tests (both longitudinal and transverse) intended to measure the percent elongation of the alloy, bending tests, and the like. It will be understood by those reviewing this disclosure that whether an alloy is sufficiently formable for a particular application will depend on a variety of factors, including the size and shape of the device to be formed, the temperature at which such forming will be performed, and other factors. For example, in the case of implantable medical devices such as the IMD 10 illustrated in FIGURE 1, a subjective determination may be made as to the formability of various alloys. For such applications, preferred alloys for such an application will have a percent elongation at a temperature of approximately 25°C of greater than approximately 13 percent and may be rolled into sheets having thicknesses of between approximately 0.007 and 0.016 inches (0.1778 - 0.4064 mm) (e.g., between approximately 0.007 and 0.012 inches 0.1778 - 0.3048 mm)).

According to an exemplary embodiment, the alloy has a composition of Ti-4.5Al-3V-2Fe-2Mo-0.15O. One example of such an alloy is commercially available from JFE Steel Corporate of Chiba, Japan under the trade name SP-700. Based on known properties of this alloy, it is believed that such a material will be sufficiently biocompatible to allow its use in implantable medical devices such as INS devices, while also providing enhanced power coupling and recharging efficiency, improved telemetry distance, reduced heating effects during magnetic resonance imaging (MRI), and improved tolerance for compression stress as compared to other alloys such as CP Ti Grade 1 and Ti-6Al-4V alloys.

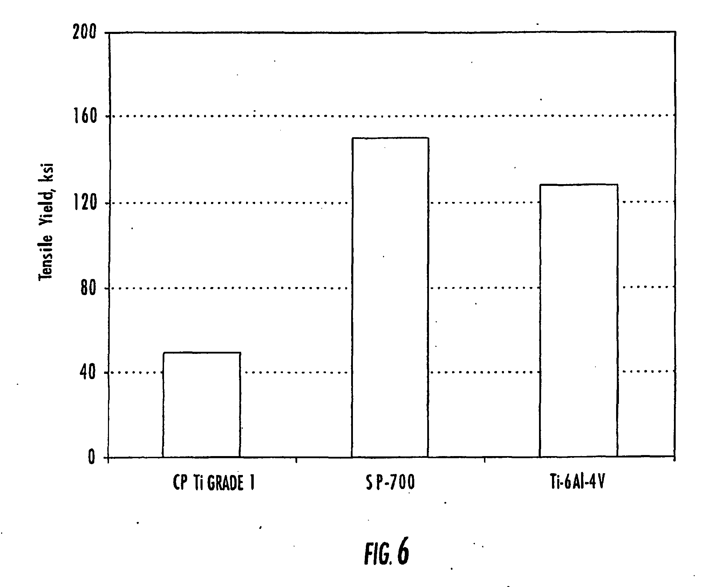

FIGURE 6 is a graph illustrating the tensile yield strength of SP-700 alloy as compared to CP Ti Grade 1 and Ti-6Al-4V alloys. As shown, the SP-700 alloy has a tensile yield strength that is approximately three times greater than CP Ti Grade 1 and also has a higher tensile yield strength than the Ti-6Al-4V alloy. It is intended that this property of the SP-700 alloy will contribute to enhanced strength of the resulting INS packaging and improved resistivity.

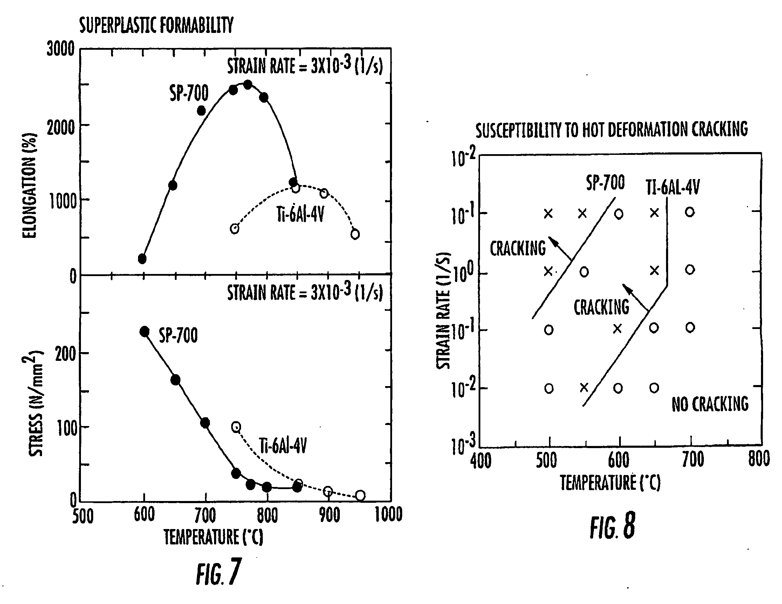

An additional advantageous feature of the SP-700 alloy is that it has a relatively small grain size that allows it to be superplastically deformed at a relatively low temperature (e.g., approximately 775°C). FIGURE 7 is a graph illustrating the relationship between percent elongation and temperature for the SP-700 alloy and the Ti-6Al-4V alloy. As evident from the graph, the SP-700 alloy is significantly more formable at lower temperatures as compared to the Ti-6Al-4V alloy. It is intended that by using the SP-700 alloy, relatively thin (< 0.4 mm) sheets of the material may be produced which may be formed into housings for INS devices that have relatively complex geometries or shapes. Additionally, as illustrated in FIGURE 8, the SP-700 alloy is less susceptible to hot deformation cracking as compared to Ti-6Al-4V alloys.

According to other exemplary embodiments, other titanium alloys may be used to form the housing 20. One such alloy is commercially available from Allvac of Albany, Oregon under the trade name ATI425 and having a composition of Ti-4Al-2.5V-1.5Fe-0.25O. According to other exemplary embodiments, titanium alloys Ti-6Al-2Sn-4Zr-2Mo (Ti6242), Ti-3Al-2.5V (Grade 9)
are used to form the housing.

**Table 2**

| Alloy | Tensile Yield Strength (ksi) | Percent Elongatio n | Resistivity (µmOhm-cm) | Bend Ratio (R/t | Cold Rolling Reduction Limit (%) | Formability | Sheet Thickness (inches) |
|---|---|---|---|---|---|---|---|
| CP Ti Gr 1 | 32 | 25 | 45 | 1.5 | >80 | Excellent | 0.001 |
| Ti-6Al-4V | 130 | 11 | 168 | 4 | 20 | Not good | 0.016 |
| SP-700 | 134 | 21 | 164 | 2.1 | 58-69 | Good | 0.007 |
| ATI425 | 127 | 18 | | 2.1 | Similar to SP-700 | Good | 0.007 |
| Ti-3Al-2.5V (Gr 9) | 72 | 20 | 125 | 2.1 | 60-80 | Very Good | 0.01 |
| | | | | | | | |
| Target | 120 | > 12 | 125-200 | < 3 | >50 | - | < 0.012 |

Table 2 illustrates properties of the titanium alloys described above as compared to CP Ti Grade 1 and Ti-6Al-4V alloys. As shown in Table 2, while both the SP-700 and Ti-6Al-4V alloys have higher resistivities and tensile yield strengths as compared to CP Ti Grade 1, the SP-700 alloy has significantly better percent elongation, formability (a subjective measure), and cold rolling reduction limit as compared to the Ti-6Al-4V alloy, which allows it to be formed into sheets that are significantly thinner than those formed from the Ti-6Al-4V alloy (e.g., sheets having a thickness of 0.0,07 in (0.1778 mm) as opposed to sheets having a thickness of 0.016 in (0.4064 mm)). It is also notable that the ATI425 alloy , has similar characteristics as the SP-700 alloy, and is expected to perform similarly from a formability and cold rolling reduction limit.

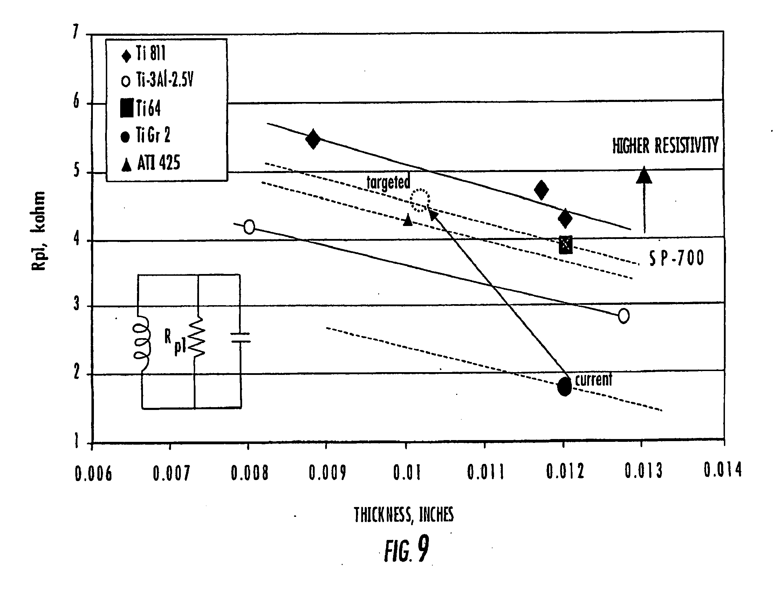

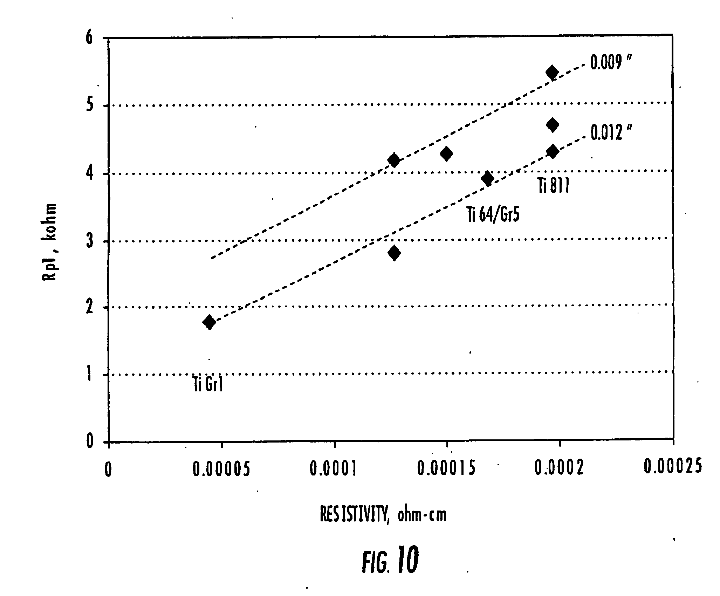

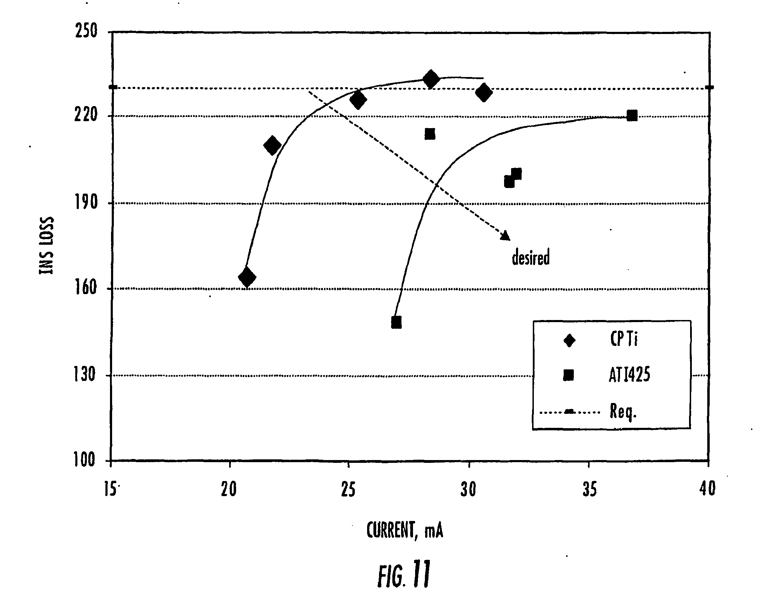

FIGURE 9 illustrates the relationship between the effective parallel coil resistance (Rpl) and housing thickness for housings formed from various titanium alloys at a charging frequency of 175 kHz. As shown in FIGURE 10, the effective parallel coil resistance tends to decrease with increasing housing thickness. As shown in FIGURE 11, the effective parallel coil resistance also tends to increase with increasing resistivity; thus, one would expect that alloys having relatively high resistivities will also exhibit relatively good effective parallel coil resistance.

By using high resistivity alloys and a thinner gauge sheet of material, an effective parallel coil resistance of as much as three times that of CP Ti Grade 1 alloy may be achieved. Additionally, it should be noted that housings formed from the ATI425 alloy exhibit effective parallel coil resistance that is less than those formed from the Ti-6Al-4V alloy. Because the resistivity of the SP-700 alloy is comparable to that of the Ti-6Al-4V alloy, it is expected that the effective parallel coil resistance of these two alloys will also be similar.

FIGURE 11 illustrates the relationship between current and the INS loss for devices having housings made using two different alloys. As illustrated in FIGURE 11, the devices having housings formed from the ATI425 alloy exhibit less INS loss at given amounts of current as compared to those having housings formed from the CP Ti Grade 1 alloy. As a result, it is expected that housings formed from ATI425 alloys would exhibit improved charging efficiency as compared to those formed from Ti Grade 1 alloys.

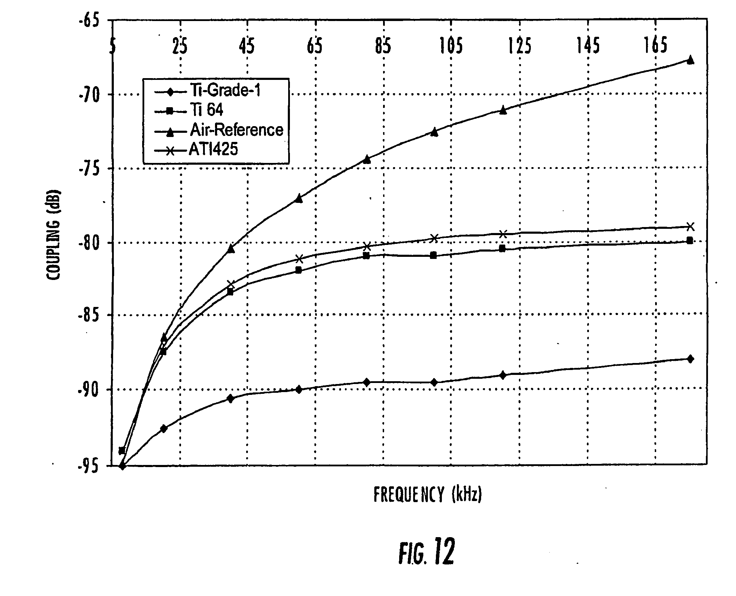

FIGURE 12 illustrates the relationship between charging frequency and coil coupling for devices having housings formed from various titanium alloys. The coil voltage coupling from 8 kHz to 175 kHz was first measured for a ten centimeter air gap between the charging coils for reference purposes, after which the coil voltage coupling was measured using sheet material inserted between the inductive charging coils.

As illustrated in FIGURE 12, there is an approximately +27 decibel voltage coupling increase in air when the frequency is increased from 8 kHz to 175 kHz. In contrast, when a 0.012 inch (0.3048 mm) thick sheet of CP Ti Grade 1 is inserted between the induction coils, the increase in voltage coupling is only +7 decibels. As shown in FIGURE 12, higher resistivity alloys (e.g., Ti-6Al-4V, ATI425) demonstrated higher coupling efficiencies at higher frequencies. Devices having housings formed from the ATI425 alloy provided better coil coupling as compared to those having housings formed from Ti-6Al-4V at all frequencies. Such improved coil coupling may be expected to provide improved telemetry distance as compared to devices having housings formed from CP Ti Grade 1 alloys. For example, it has been determined that the telemetry distance that may be achieved using an SP-700 alloy housing exceeds that which may be achieved using a CP Ti Grade 1 alloy by approximately 30 cm.

It is expected that various advantages may be obtained by utilizing the alloys in claim 1. to form housings for implantable medical devices. For example, devices using such alloys may have improved recharging efficiency (e.g., such devices may have an approximately 8 dB power coupling increase for recharging frequencies over 50 kHz as compared to devices having housings formed from CP Ti Grade 1 alloy). This may result in an approximately tenfold increase in power transfer.

Because the alloys used to form the housings have improved tensile strength as compared to Ti Grade 1, the housings may be formed having thinner walls, which contribute to the improved telemetry distance and power coupling efficiency. According to an exemplary embodiment, the housing is formed from an alloy selected from the group consisting of SP-700, ATI425, Ti-3Al-2.5V, Ti6242 and has a thickness of between approximately 0.01 and 0.016 inches (0.254 - 0.4064 mm). According to other exemplary embodiments, the housing walls have thicknesses of between approximately 0.01 and 0.012 inches (0.254 - 0.3048 mm).

While the various exemplary embodiments have been described in relation to an implantable neurological stimulation device, it should be noted that titanium alloys such as those described herein may be used in connection with other implantable medical devices as well. For example, the titanium alloys described herein may be used as a diaphragm material for an implantable pressure sensor (e.g., which may be integrated into a drug pump as a catheter diagnostic aid). Because diaphragms made from CP titanium (Ti) Grade 1 must be relatively thick, the signal to noise ratio of the sensor is relatively high. While the use of Ti-6Al-4V will help increase both strength and the signal to noise ratio, such an alloy is not available in sheets thin enough for practical sensors. The titanium alloys described herein offer the possibility of a sensor with higher signal to noise ratios that also meet the overpressure requirements for such components. The titanium alloys described herein may also find utility as housing materials for cardiac pacemakers.

As another example of a possible application, the titanium alloys described herein may be used as an outer shield for a drug pump. One advantageous feature of utilizing the alloys described herein is that it is believed that the use of such alloys may improve the efficiency of telemetry of the pump in a similar manner to that described above in the context of implantable INS devices.

It is important to note that the construction and arrangement of the implantable medical device as shown in the various exemplary embodiments is illustrative only. Although only a few embodiments of the present inventions have been described in detail in this disclosure, those skilled in the art who review this disclosure will readily appreciate that many modifications are possible without materially departing from the novel teachings and advantages of the subject matter recited in the claims. Accordingly, all such modifications are intended to be included within the scope of the present invention as defined in the appended claims. The order or sequence of any process or method steps may be varied or resequenced according to alternative embodiments. Other substitutions, modifications, changes and omissions may be made in the design, operating conditions and arrangement of the various exemplary embodiments without departing from the scope of the present invention as expressed in the appended claims.

## Claims

1. An implantable medical device (10) comprising:
a housing (20) formed from a titanium alloy selected from the group consisting of Ti-4.5Al-3V-2Fe-2Mo-0.15O, Ti-4Al-2.5V-1.5Fe-0.25O, Ti-6Al-2Sn-4Zr-2Mo, Ti-3Al-2.5V, and combinations thereof, wherein the implantable medical device is rechargeable.

2. The implantable medical device (10) as recited in Claim 1, wherein the implantable medical device is selected from the group consisting of an implantable neurological stimulation device and a cardiac pacemaker.

3. The implantable medical device (10) as recited in Claim 1, further comprising an electrically conductive coil provided within the housing (20) to facilitate inductive charging of the implantable medical device (10).

4. The implantable medical device (10) as recited in any of Claims 1 through 3, wherein the titanium alloy comprises a Ti-4.SAl-3V-2Fe-2Mo-0.15O alloy.

5. The implantable medical device (10) as recited in any of Claims 1 through 3, wherein the titanium alloy comprises a Ti-4Al-2.5V-1.5Fe-0.25O alloy.

6. The implantable medical device (10) as recited in any of Claims 1 through 3, wherein the titanium alloy comprises a Ti-6Al-2Sn-4Zr-2Mo alloy.

7. The implantable medical device (10) as recited in any of Claims 1 through 3, wherein the titanium alloy comprises a Ti-3Al-2.5V alloy.

8. The implantable medical device (10) as recited in any of Claims 1 through 3, wherein the housing (20) has improved telemetry characteristics as compared to a housing made of commercial pure titanium Grade 1.

9. The implantable medical device (10) as recited in any of Claims 1 through 3, wherein the titanium alloy has a tensile yield strength between 70 ksi and 150 ksi and a resistivity between 100 µmΩ-cm and 210 µmΩ-cm.

10. The implantable medical device (10) as recited in any of Claims 1 through 3, wherein the housing (20) comprises walls having a thickness of between 0.007 and 0.016 inches (0.1778 - 0.4064 mm).

11. The implantable medical device (10) as recited in any of Claims 1 through3, wherein at least a portion of the housing (20) has a radius of curvature of between 0.1 and 2.5 millimeters.

12. The implantable medical device (10) as recited in any of Claims 1 through 3, wherein the titanium alloy exhibits greater formability as compared to a Ti-6Al-4V alloy.

## Patentansprüche

1. Implantierbare medizinische Vorrichtung (10) mit:
einem Gehäuse (20), das aus einer Titanlegierung gebildet ist, welche ausgewählt ist aus der Gruppe bestehend aus Ti-4,5Al-3V-2Fe-2Mo-0,15O, Ti-4Al-2,5V-1,5Fe-0,250, Ti-6Al-2Sn-4Zr-2Mo, Ti-3Al-2,5V und Kombinationen daraus, wobei die implantierbare medizinische Vorrichtung wiederaufladbar ist.

2. Implantierbare medizinische Vorrichtung (10) nach Anspruch 1, wobei die implantierbare medizinische Vorrichtung ausgewählt ist aus der Gruppe bestehend aus einer implantierbaren neurologischen Stimulationsvorrichtung und einem Herzschrittmacher.

3. Implantierbare medizinische Vorrichtung (10) nach Anspruch 1, ferner mit einer elektrisch leitfähigen Spule bzw. Wicklung, die innerhalb des Gehäuses (20) bereitgestellt ist, um ein induktives Aufladen der implantierbaren medizinischen Vorrichtung (10) zu ermöglichen.

4. Implantierbare medizinische Vorrichtung (10) nach einem der Ansprüche 1 bis 3, wobei die Titanlegierung eine Ti-4,5Al-3V-2Fe-2Mo-0,15O Legierung umfasst.

5. Implantierbare medizinische Vorrichtung (10) nach einem der Ansprüche 1 bis 3, wobei die Titanlegierung eine Ti-4Al-2,5V-1,5Fe-0,25O Legierung umfasst.

6. Implantierbare medizinische Vorrichtung (10) nach einem der Ansprüche 1 bis 3, wobei die Titanlegierung eine Ti-6Al-2Sn-4Zr-2Mo Legierung umfasst.

7. Implantierbare medizinische Vorrichtung (10) nach einem der Ansprüche 1 bis 3, wobei die Titanlegierung eine Ti-3Al-2,5V Legierung umfasst.

8. Implantierbare medizinische Vorrichtung (10) nach einem der Ansprüche 1 bis 3, wobei das Gehäuse (20) verbesserte Telemetriecharakteristika im Vergleich zu einem Gehäuse, das aus herkömmlichem reinem Titan Klasse 1 hergestellt ist, hat.

9. Implantierbare medizinische Vorrichtung (10) nach einem der Ansprüche 1 bis 3, wobei die Titanlegierung eine Dehn- bzw. Streckgrenze zwischen 70 ksi und 150 ksi und einen spezifischen Widerstand zwischen 100 µmΩ-cm und 210 µmΩ-cm aufweist .

10. Implantierbare medizinische Vorrichtung (10) nach einem der Ansprüche 1 bis 3, wobei das Gehäuse (20) Wände mit einer Dicke zwischen 0,007 und 0,16 Zoll (0,1778 bis 0,4064 mm) aufweist.

11. Implantierbare medizinische Vorrichtung (10) nach einem der Ansprüche 1 bis 3, wobei zumindest ein Teil des Gehäuses (20) einen Krümmungsradius zwischen 0,1 und 2,5 mm aufweist.

12. Implantierbare medizinische Vorrichtung (10) nach einem der Ansprüche 1 bis 3, wobei die Titanlegierung eine im Vergleich zu einer Ti-6Al-4V Legierung größere Formfähigkeit zeigt.

## Revendications

1. Dispositif médical implantable (10) comportant :
un boîtier (20) formé à partir d'un alliage en titane sélectionné à partir du groupe constitué de Ti-4,5Al-3V-2Fe-2Mo-0,15O, Ti-4Al-2,5V-1,5Fe-0,25O, Ti-6Al-2Sn-4Zr-2Mo, Ti-3Al-2,5V, et des combinaisons de ceux-ci, dans lequel le dispositif médical implantable est rechargeable.

2. Dispositif médical implantable (10) selon la revendication 1, dans lequel le dispositif médical implantable est sélectionné à partir du groupe constitué d'un dispositif de stimulation neurologique implantable et d'un stimulateur cardiaque.

3. Dispositif médical implantable (10) selon la revendication 1, comportant en outre une bobine électriquement conductrice agencée dans le boîtier (20) pour faciliter un chargement inductif du dispositif médical implantable (10).

4. Dispositif médical implantable (10) selon l'une quelconque des revendications 1 à 3, dans lequel l'alliage de titane comporte un alliage Ti-4,5Al-3V-2Fe-2Mo-0,150.

5. Dispositif médical implantable (10) selon l'une quelconque des revendications 1 à 3, dans lequel l'alliage de titane comporte un alliage Ti-4Al-2,5V-1,5Fe-0,250.

6. Dispositif médical implantable (10) selon l'une quelconque des revendications 1 à 3, dans lequel l'alliage de titane comporte un alliage Ti-6Al-2Sn-4Zr-2Mo.

7. Dispositif médical implantable (10) selon l'une quelconque des revendications 1 à 3, dans lequel l'alliage de titane comporte un alliage Ti-3Al-2,5V.

8. Dispositif médical implantable (10) selon l'une quelconque des revendications 1 à 3, dans lequel le boîtier (20) a des caractéristiques de télémesure améliorées comparativement à un boîtier constitué de titane pur commercial de grade 1.

9. Dispositif médical implantable (10) selon l'une quelconque des revendications 1 à 3, dans lequel l'alliage de titane a une résistance à la traction comprise entre 70ksi et 150 ksi et une résistivité comprise entre 100 µmΩ-cm et 210 µmΩ-cm.

10. Dispositif médical implantable (10) selon l'une quelconque des revendications 1 à 3, dans lequel le boîtier (20) comporte des parois ayant une épaisseur comprise entre 0,007 et 0,016 pouce (0,1778 - 0,4064 mm).

11. Dispositif médical implantable (10) selon l'une quelconque des revendications 1 à 3, dans lequel au moins une partie du boîtier (20) a un rayon de courbure compris entre 0,1 et 2,5 millimètres.

12. Dispositif médical implantable (10) selon l'une quelconque des revendications 1 à 3, dans lequel l'alliage de titane présente une formabilité supérieure comparativement à celle d'un alliage Ti-6Al-4V.
